(19) Europäisches Patentamt
European Patent Office
Office européen des brevets

(11) Veröffentlichungsnummer: **0 043 419**
**B1**

(12) # EUROPÄISCHE PATENTSCHRIFT

(45) Veröffentlichungstag der Patentschrift: **14.03.84**

(51) Int. Cl.³: **C 07 D 233/60,** C 07 D 249/08, A 61 K 31/41

(21) Anmeldenummer: **81103337.2**

(22) Anmeldetag: **04.05.81**

(54) **Antimykotisches Mittel.**

(30) Priorität: **16.05.80 DE 3018865**

(43) Veröffentlichungstag der Anmeldung: **13.01.82 Patentblatt 82/2**

(45) Bekanntmachung des Hinweises auf die Patenterteilung: **14.03.84 Patentblatt 84/11**

(84) Benannte Vertragsstaaten: **BE CH DE FR GB IT LI NL SE**

(56) Entgegenhaltungen:
**EP - A - 0 005 250**
**EP - A - 0 011 768**
**EP - A - 0 015 756**
**DE - A - 2 623 129**
**FR - A - 2 299 807**
**FR - A - 2 370 432**
**FR - A - 2 399 803**
**GB - A - 1 522 848**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Holmwood, Graham, Dr., Katernbergerstrasse 184, D-5600 Wuppertal 1 (DE)**
Erfinder: **Büchel, Karl Heinz, Prof. Dr., Dabringhausener Strasse 42, D-5093 Burscheid (DE)**
Erfinder: **Plempel, Manfred, Dr., Pahlkestrasse 5, D-5600 Wuppertal 1 (DE)**
Erfinder: **Haller, Ingo, Dr., Viktoriastrasse 99, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 043 419 B1

Antimykotisches Mittel

Die Erfindung betrifft die Verwendung von neuen 1-Hydroxyethyl-azol-Derivaten als antimykotische Mittel.

Es ist bereits bekanntgeworden, daß tertiäre Imidazolylalkohole, wie z. B. das 1,2-Bis(4-chlorphenyl)-3-(imidazol-1-yl)-2-propanol, gute antimykotische Eigenschaften aufweisen (vergleiche DE-OS 2 623 129). Deren Wirkung ist jedoch nicht immer voll befriedigend.

Weiterhin ist bekanntgeworden, daß 1-(β-Aryl)-ethylimidazol-Derivate, wie insbesondere das 1-[2,4-Dichlor-β-(2,4-dichlorbenzyloxy)-phenethyl[-imidazolnitrat (Miconazol), gute antimykotische Wirkung zeigen (vgl. DE-AS 1 940 388). Diese Wirkung ist jedoch in vivo, wie insbesondere gegen Candida, nicht immer befriedigend.

Es wurde nun gefunden, daß die 1-Hydroxyethyl-azol-Derivate der allgemeinen Formel I

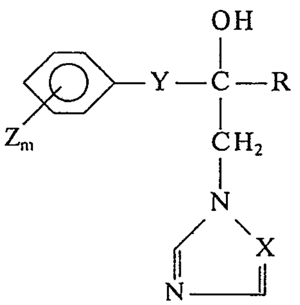

(I)

in welcher

R für geradkettiges oder verzweigtes Alkyl mit 1–4 Kohlenstoffatomen, gegebenenfalls durch Alkyl mit 1–2 Kohlenstoffatomen substituiertes Cycloalkyl mit 3–7 Kohlenstoffatomen sowie gegebenenfalls einfach oder mehrfach, gleich oder verschieden durch Halogen, Alkyl mit 1–4 Kohlenstoffatomen oder Halogenalkyl mit 1–2 Kohlenstoffatomen und 1–5 gleichen oder verschiedenen Halogenatomen substituiertes Phenyl steht,

X für ein Stickstoffatom oder die –CH-Gruppe steht,

Y für die Gruppierungen $-OCH_2-$, $-CH_2CH_2-$ oder $-CH=CH-$ steht,

Z für Halogen, geradkettiges oder verzweigtes Alkyl mit 1–4 Kohlenstoffatomen, Cycloalkyl mit 5–7 Kohlenstoffatomen, Alkoxy und Alkylthio mit jeweils 1–4 Kohlenstoffatomen, Halogenalkyl und Halogenalkoxy sowie Halogenalkylthio mit jeweils 1–2 Kohlenstoffatomen und 1–5 gleichen oder verschiedenen Halogenatomen sowie für jeweils gegebenenfalls durch Halogen oder Alkyl mit 1–4 Kohlenstoffatomen substituiertes Phenyl, Phenoxy, Phenylalkyl oder Phenylalkoxy mit jeweils 1–2 Kohlenstoffatomen im Alkylteil steht und

m für die Zahlen 0, 1, 2 oder 3 steht,

sowie deren physiologisch verträglichen Säureadditions-Salze gute antimikrobielle, insbesondere antimykotische Eigenschaften aufweisen.

Die Verbindungen der allgemeinen Formel I besitzen ein asymmetrisches Kohlenstoffatom und können deshalb in den beiden optischen Isomerenformen anfallen. Wenn Y für die Gruppierung $-CH=CH-$ steht, können die Verbindungen der allgemeinen Formel I zusätzlich in zwei geometrischen Isomerenformen vorliegen. Die Erfindung betrifft sowohl die Isomerengemische als auch die einzelnen Isomeren.

Überraschenderweise zeigen die erfindungsgemäßen 1-Hydroxyethyl-azol-Derivate ein besseres antimykotisches Wirkungsspektrum als das aus dem Stand der Technik bekannte 1,2-Bis-(4-chlorphenyl)-3-(imidazol-1-yl)-2-propanol sowie insbesondere eine bessere, therapeutisch nutzbare in-vivo-Wirksamkeit als das aus dem Stand der Technik bekannte 1-[2,4-Dichlor-β-(2,4-dichlorbenzyloxy)-phenethyl]-imidazolnitrat, welches ein anerkannt gutes Mittel gleicher Wirkungsrichtung ist. Die erfindungsgemäße Verwendung der Wirkstoffe stellt somit eine wertvolle Bereicherung der Pharmazie dar. Besonders bevorzugt sind diejenigen Verbindungen der allgemeinen Formel I, in denen R für tert.-Butyl, Isopropyl oder Methyl steht, für jeweils gegebenenfalls durch Methyl substituiertes Cyclopropyl, Cyclopentyl oder Cyclohexyl steht sowie für gegebenenfalls einfach oder zweifach, gleich oder verschieden durch Fluor, Chlor, Methyl oder Trifluormethyl substituiertes Phenyl steht; Z für Fluor, Chlor, Brom, Methyl, tert.-Butyl, Cyclohexyl, Methoxy, Methylthio, Trifluormethyl, Trifluormethyl, Trifluormethoxy, Trifluormethylthio sowie für jeweils gegebenenfalls einfach oder zweifach, gleich oder verschieden durch Fluor, Chlor und Methyl substituiertes Phenyl, Phenoxy, Benzyl oder Benzyloxy steht und X, Y sowie der Index m die in der Erfindungsdefinition angegebene Bedeutung haben.

Im einzelnen seien außer den bei den Herstellungsbeispielen genannten Verbindungen folgende Verbindungen der allgemeinen Formel I genannt:

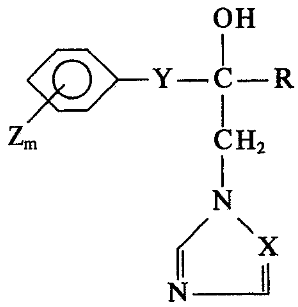

(I)

| $Z_m$ | Y | R | X |
|---|---|---|---|
| 4- 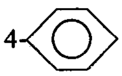 | $-O-CH_2-$ | $-C(CH_3)_3$ | N(CH) |
| 4- 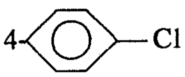 | desgl. | desgl. | desgl. |
| 4-O — 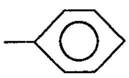 | desgl. | desgl. | desgl. |
| 4-O — 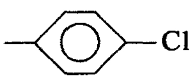 | desgl. | desgl. | desgl. |
| $4-CH_2$— 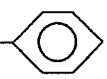 | desgl. | desgl. | desgl. |
| $4-CH_2$— 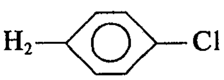 | desgl. | desgl. | desgl. |
| $4-O-CH_2$— 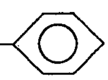 | desgl. | desgl. | desgl. |
| $4-O-CH_2$— 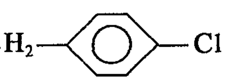 | desgl. | desgl. | desgl. |
| $3,4-Cl_2$ | desgl. | desgl. | desgl. |
| $4-CF_3$ | desgl. | desgl. | desgl. |
| $4-OCF_3$ | desgl. | desgl. | desgl. |
| $4-SCF_3$ | desgl. | desgl. | desgl. |
| $4-SCH_3$ | desgl. | desgl. | desgl. |
| $4-C(CH_3)_3$ | desgl. | desgl. | desgl. |
| 4- 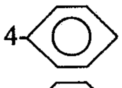 | $-O-CH_2$ | 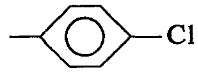 | N(CH) |
| 4- 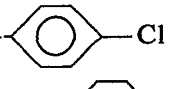 | desgl. | desgl. | desgl. |
| 4-O — 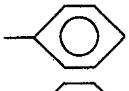 | desgl. | desgl. | desgl. |
| 4-O — 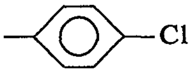 | desgl. | desgl. | desgl. |

(Fortsetzung)

| $Z_m$ | Y | R | X |
|---|---|---|---|
| | —O—$CH_2$ | 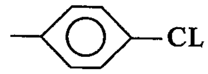 | N(CH) |
| 4-$CH_2$— 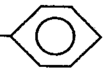 | desgl. | desgl. | desgl. |
| 4-$CH_2$— 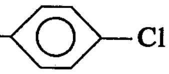 | desgl. | desgl. | desgl. |
| 4-O—$CH_2$— 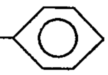 | desgl. | desgl. | desgl. |
| 4-O—$CH_2$— 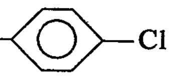 | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 4- 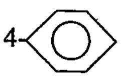 | —O—$CH_2$— | —$CH(CH_3)_2$ | N(CH) |
| 4- 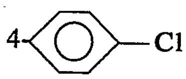 | desgl. | desgl. | desgl. |
| 4-O— 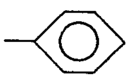 | desgl. | desgl. | desgl. |
| 4-O— 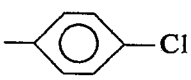 | desgl. | desgl. | desgl. |
| 4-$CH_2$— 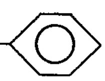 | desgl. | desgl. | desgl. |
| 4-$CH_2$— 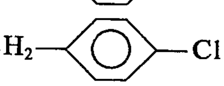 | desgl. | desgl. | desgl. |
| 4-O—$CH_2$— 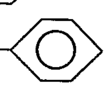 | desgl. | desgl. | desgl. |
| 4-O—$CH_2$— 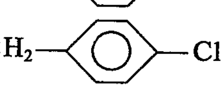 | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |

(Fortsetzung)

| $Z_m$ | Y | R | X |
|---|---|---|---|
| 4-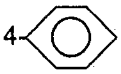 | —O—$CH_2$— | 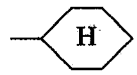 | N(CH) |
| 4-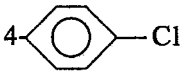 | desgl. | desgl. | desgl. |
| 4-O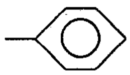 | desgl. | desgl. | desgl. |
| 4-O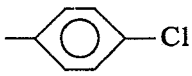 | desgl. | desgl. | desgl. |
| 4-$CH_2$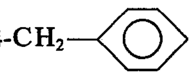 | desgl. | desgl. | desgl. |
| 4-$CH_2$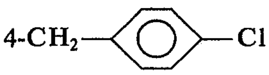 | desgl. | desgl. | desgl. |
| 4-O—$CH_2$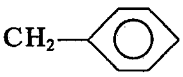 | desgl. | desgl. | desgl. |
| 4-O—$CH_2$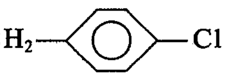 | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 4-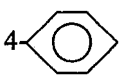 | —O—$CH_2$— | 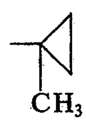 | N(CH) |
| 4-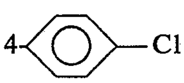 | desgl. | desgl. | desgl. |
| 4-O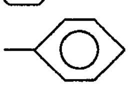 | desgl. | desgl. | desgl. |
| 4-O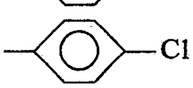 | desgl. | desgl. | desgl. |
| 4-$CH_2$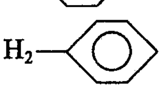 | desgl. | desgl. | desgl. |
| 4-$CH_2$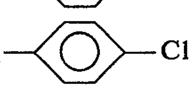 | desgl. | desgl. | desgl. |
| 4-O—$CH_2$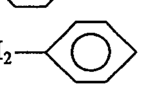 | desgl. | desgl. | desgl. |
| 4-O—$CH_2$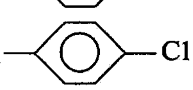 | desgl. | desgl. | desgl. |
| 3,4-$Cl_2$ | desgl. | desgl. | desgl. |
| 4-$CF_3$ | desgl. | desgl. | desgl. |

(Fortsetzung)

| $Z_m$ | Y | R | X |
|---|---|---|---|
| | $—O\,CH_2—$ | 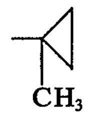 | N(CH) |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 4-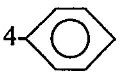 | $—CH_2—CH_2—$ | $—C(CH_3)_3$ | N(CH) |
| 4-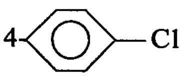 | desgl. | desgl. | desgl. |
| 4-O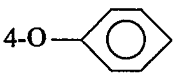 | desgl. | desgl. | desgl. |
| 4-O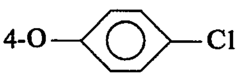 | desgl. | desgl. | desgl. |
| 4-$CH_2$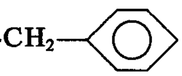 | desgl. | desgl. | desgl. |
| 4-$CH_2$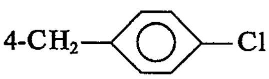 | desgl. | desgl. | desgl. |
| 4-O—$CH_2$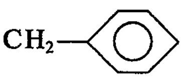 | desgl. | desgl. | desgl. |
| 4-O—$CH_2$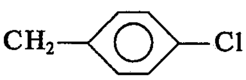 | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 4-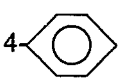 | $—CH_2—CH_2—$ | 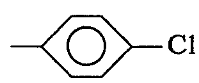 | N(CH) |
| 4-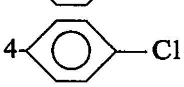 | desgl. | desgl. | desgl. |
| 4-O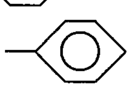 | desgl. | desgl. | desgl. |
| 4-O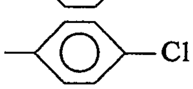 | desgl. | desgl. | desgl. |
| 4-$CH_2$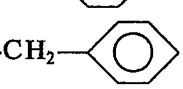 | desgl. | desgl. | desgl. |

(Fortsetzung)

| $Z_m$ | Y | R | X |
|---|---|---|---|
| | $—CH_2—CH_2—$ | 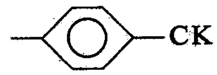 | N(CH) |
| 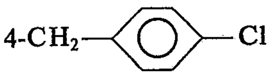 | desgl. | desgl. | desgl. |
| 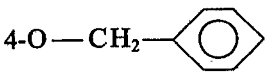 | desgl. | desgl. | desgl. |
| 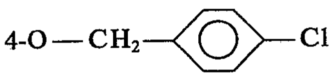 | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 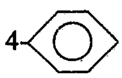 | $—CH_2—CH_2—$ | $—CH(CH_3)_2$ | N(CH) |
| 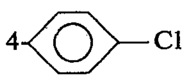 | desgl. | desgl. | desgl. |
| 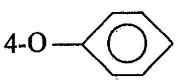 | desgl. | desgl. | desgl. |
| 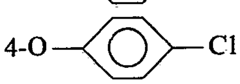 | desgl. | desgl. | desgl. |
| 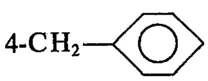 | desgl. | desgl. | desgl. |
| 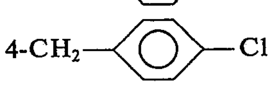 | desgl. | desgl. | desgl. |
| 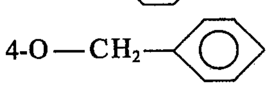 | desgl. | desgl. | desgl. |
| 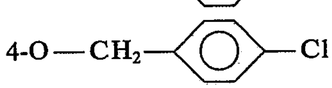 | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |

(Fortsetzung)

| $Z_m$ | Y | R | X |
|---|---|---|---|
| 4-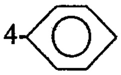 | $-CH_2-CH_2-$ | 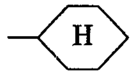 | N(CH) |
| 4-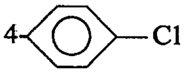 | desgl. | desgl. | desgl. |
| 4-O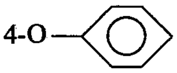 | desgl. | desgl. | desgl. |
| 4-O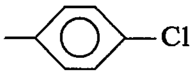 | desgl. | desgl. | desgl. |
| 4-$CH_2$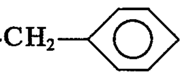 | desgl. | desgl. | desgl. |
| 4-$CH_2$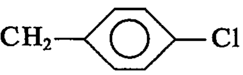 | desgl. | desgl. | desgl. |
| 4-O—$CH_2$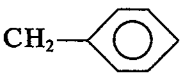 | desgl. | desgl. | desgl. |
| 4-O—$CH_2$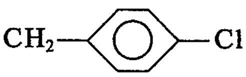 | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 4-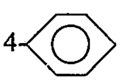 | $-CH_2-CH_2-$ | 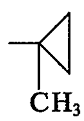 | N(CH) |
| 4-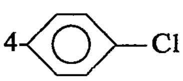 | desgl. | desgl. | desgl. |
| 4-O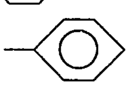 | desgl. | desgl. | desgl. |
| 4-O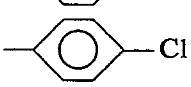 | desgl. | desgl. | desgl. |
| 4-$CH_2$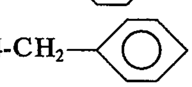 | desgl. | desgl. | desgl. |
| 4-$CH_2$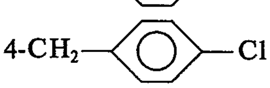 | desgl. | desgl. | desgl. |
| 4-O—$CH_2$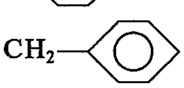 | desgl. | desgl. | desgl. |
| 4-O—$CH_2$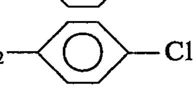 | desgl. | desgl. | desgl. |
| 3,4-$Cl_2$ | desgl. | desgl. | desgl. |
| 4-$CF_3$ | desgl. | desgl. | desgl. |

(Fortsetzung)

| $Z_m$ | Y | R | X |
|---|---|---|---|
| | $—CH_2—CH_2—$ | 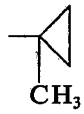 | N(CH) |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 4-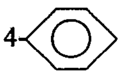 | $—CH{=}CH—$ | $—C(CH_3)_3$ | N(CH) |
| 4-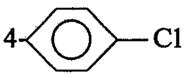 | desgl. | desgl. | desgl. |
| 4-O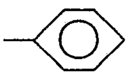 | desgl. | desgl. | desgl. |
| 4-O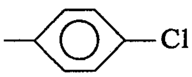 | desgl. | desgl. | desgl. |
| 4-$CH_2$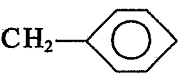 | desgl. | desgl. | desgl. |
| 4-$CH_2$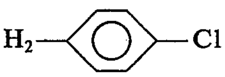 | desgl. | desgl. | desgl. |
| 4-O — $CH_2$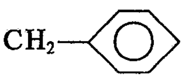 | desgl. | desgl. | desgl. |
| 4-O — $CH_2$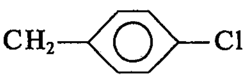 | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 4-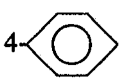 | $—CH{=}CH—$ | 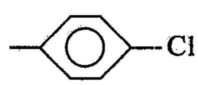 | N(CH) |
| 4-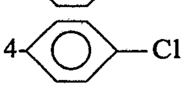 | desgl. | desgl. | desgl. |
| 4-O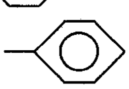 | desgl. | desgl. | desgl. |
| 4-O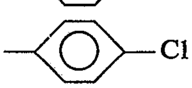 | desgl. | desgl. | desgl. |
| 4-$CH_2$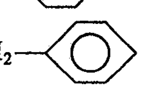 | desgl. | desgl. | desgl. |

(Fortsetzung)

| $Z_m$ | Y | R | X |
|---|---|---|---|
| | $—CH{=}CH—$ | 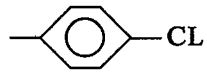 | N(CH) |
| 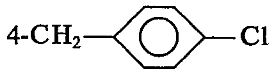 | desgl. | desgl. | desgl. |
| 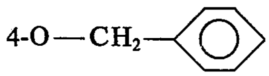 | desgl. | desgl. | desgl. |
| 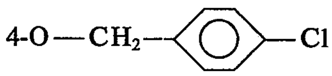 | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 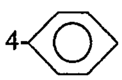 | $—CH{=}CH—$ | $—CH(CH_3)_2$ | N(CH) |
| 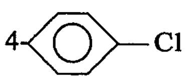 | desgl. | desgl. | desgl. |
| 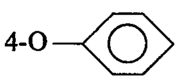 | desgl. | desgl. | desgl. |
| 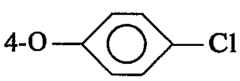 | desgl. | desgl. | desgl. |
| 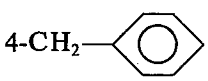 | desgl. | desgl. | desgl. |
| 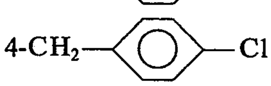 | desgl. | desgl. | desgl. |
| 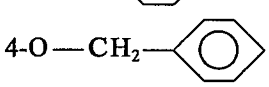 | desgl. | desgl. | desgl. |
| 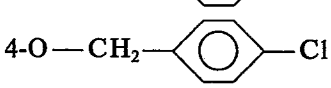 | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |

(Fortsetzung)

| $Z_m$ | Y | R | X |
|---|---|---|---|
| 4-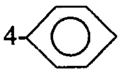 | —CH=CH— | 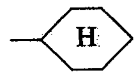 | N(CH) |
| 4-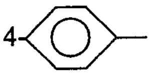Cl | desgl. | desgl. | desgl. |
| 4-O—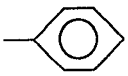 | desgl. | desgl. | desgl. |
| 4-O—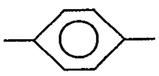Cl | desgl. | desgl. | desgl. |
| 4-$CH_2$—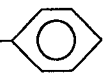 | desgl. | desgl. | desgl. |
| 4-$CH_2$—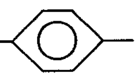Cl | desgl. | desgl. | desgl. |
| 4-O—$CH_2$—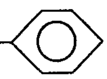 | desgl. | desgl. | desgl. |
| 4-O—$CH_2$—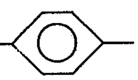Cl | desgl. | desgl. | desgl. |
| 3,4-$Cl_2$ | desgl. | desgl. | desgl. |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 4-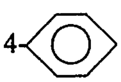 | —CH=CH— | 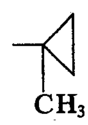 | N(CH) |
| 4-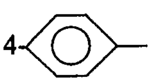Cl | desgl. | desgl. | desgl. |
| 4-O—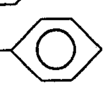 | desgl. | desgl. | desgl. |
| 4-O—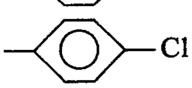Cl | desgl. | desgl. | desgl. |
| 4-$CH_2$—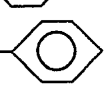 | desgl. | desgl. | desgl. |
| 4-$CH_2$—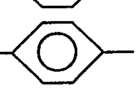Cl | desgl. | desgl. | desgl. |
| 4-O—$CH_2$—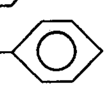 | desgl. | desgl. | desgl. |
| 4-O—$CH_2$—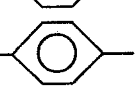Cl | desgl. | desgl. | desgl. |
| 3,4-$Cl_2$ | desgl. | desgl. | desgl. |

(Fortsetzung)

| $Z_m$ | Y | R | X |
|---|---|---|---|
| | $—CH{=}CH—$ | 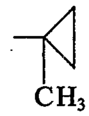 | N(CH) |
| 4-$CF_3$ | desgl. | desgl. | desgl. |
| 4-$OCF_3$ | desgl. | desgl. | desgl. |
| 4-$SCF_3$ | desgl. | desgl. | desgl. |
| 4-$SCH_3$ | desgl. | desgl. | desgl. |
| 4-$C(CH_3)_3$ | desgl. | desgl. | desgl. |
| 4-Cl | $—O—CH_2—$ | $—CH(CH_3)_2$ | N(CH) |
| 4-F | desgl. | desgl. | desgl. |
| 4-$CH_3$ | desgl. | desgl. | desgl. |
| 4-Cl | $—O—CH_2—$ | 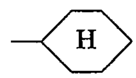 | N(CH) |
| 4-F | desgl. | desgl. | desgl. |
| 4-$CH_3$ | desgl. | desgl. | desgl. |
| 4-Cl | $—O—CH_2—$ | 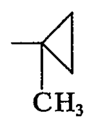 | N(CH) |
| 4-F | desgl. | desgl. | desgl. |
| 4-$CH_3$ | desgl. | desgl. | desgl. |
| 4-Cl | $—CH_2—CH_2—$ | $—CH(CH_3)_2$ | N(CH) |
| 4-F | desgl. | desgl. | desgl. |
| 4-$CH_3$ | desgl. | desgl. | desgl. |
| 4-Cl | $—CH_2—CH_2—$ | 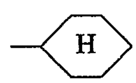 | N(CH) |
| 4-F | desgl. | desgl. | desgl. |
| 4-$CH_3$ | desgl. | desgl. | desgl. |

(Fortsetzung)

| $Z_m$ | Y | R | X |
|---|---|---|---|
| 4-Cl | $-CH_2-CH_2-$ | 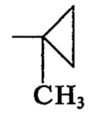 | N(CH) |
| 4-F | desgl. | desgl. | desgl. |
| $4-CH_3$ | desgl. | desgl. | desgl. |
| 4-Cl | $-CH{=}CH-$ | $-CH(CH_3)_2$ | N(CH) |
| 4-F | desgl. | desgl. | desgl. |
| $4-CH_3$ | desgl. | desgl. | desgl. |
| 4-Cl | $-CH{=}CH-$ | 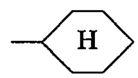 | N(CH) |
| 4-F | desgl. | desgl. | desgl. |
| $4-CH_3$ | desgl. | desgl. | desgl. |
| 4-Cl | $-CH{=}CH-$ | 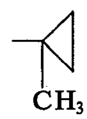 | N(CH) |
| 4-F | desgl. | desgl. | desgl. |
| $4-CH_3$ | desgl. | desgl. | desgl. |
| $2,4-Cl_2$ | $-CH_2-CH_2-$ | $-C(CH_3)_3$ | N(CH) |
| $4-CH_3$ | desgl. | desgl. | desgl. |
| 4-Cl, $2-CH_3$ | desgl. | desgl. | desgl. |
| $2,4-Cl_2$ | $-CH{=}CH-$ | $-C(CH_3)_3$ | N(CH) |
| $4-CH_3$ | desgl. | desgl. | desgl. |
| 4-Cl, $2-CH_3$ | desgl. | desgl. | desgl. |
| 4-F | $-O-CH_2-$ | $-C(CH_3)_3$ | N(CH) |
| $2-CH_3$ | $-CH_2-CH_2-$ | $-C(CH_3)_3$ | N |
| 4-F | $-CH{=}CH-$ | $-C(CH_3)_3$ | N |

Die erfindungsgemäß zu verwendenden Wirkstoffe und deren Säureadditionssalze sind noch nicht bekannt. Sie können jedoch gemäß einem eigenen Vorschlag hergestellt werden, indem man Oxirane der allgemeinen Formel II

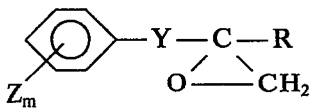

(II)

in welcher
R, Y, Z und m die oben angegebene Bedeutung haben,

mit Azolen der allgemeinen Formel III

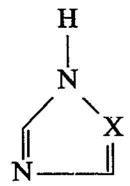

(III)

in welcher

X die oben angegebene Bedeutung hat,

in Gegenwart eines inerten organischen Lösungsmittels, wie beispielsweise Ethanol, und gegebenenfalls in Gegenwart einer Base, wie beispielsweise Natriumethylat, gegebenenfalls unter einem Druck von 1 bis 25 bar, bei Temperaturen zwischen 60 und 150°C umsetzt. Die Isolierung der Endprodukte erfolgt in allgemein üblicher Weise.

Die Oxirane der allgemeinen Formel II sind noch nicht bekannt. Sie werden erhalten, indem man entsprechende Ketone der allgemeinen Formel IV

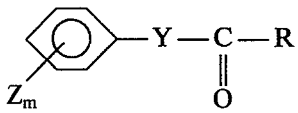

(IV)

in welcher
R, Y, Z und m die oben angegebene Bedeutung haben,

*α*) mit Dimethyloxosulfonium-methylid der Formel V

$$(CH_3)_2\overset{\delta^+}{S}O\overset{\delta^-}{C}H_2 \qquad (V)$$

in an sich bekannter Weise in Gegenwart eines Verdünnungsmittels, wie beispielsweise Dimethylsulfoxid, bei Temperaturen zwischen 20 und 80°C umsetzt [vergleiche hierzu auch die Angaben in J. Am. Chem. Soc. 87, 1363–1364 (1965)], oder

*β*) mit Trimethylsulfonium-methylsulfat der Formel VI

$$\left[(CH_3)_3\overset{(+)}{S}\right] \quad CH_3SO_4^{(-)} \qquad (VI)$$

in an sich bekannter Weise in Gegenwart eines inerten organischen Lösungsmittels, wie z. B. Acetonitril, und in Gegenwart einer Base, wie z. B. Natriummethylat, bei Temperaturen zwischen 0 bis 60°C, vorzugsweise bei Raumtemperatur, umsetzt [vergleiche auch die Angaben in Heterocycles 8, 397, (1977)].

Die so erhaltenen Oxirane der allgemeinen Formel II können gegebenenfalls ohne Isolierung direkt weiter umgesetzt werden.

Die Ketone der allgemeinen Formel IV sind bekannt (vgl. zum Beispiel DE-PS 2 201 063, DE-OS 2 705 678, DE-OS 2 737 489, Tetrahedron 1975, 31, 3, sowie C.A. 84, 73 906 u) bzw. können sie nach den dort beschriebenen Verfahren erhalten werden.

Das Dimethyloxosulfonium-methylid der Formel V ist ebenfalls bekannt und wird in situ eingesetzt, indem man Trimethyloxosulfoniumjodid mit Natriumhydrid (vgl. auch die o.g. Literaturstelle) bzw. Natriumamid umsetzt. Das Trimethylsulfonium-methylsulfat der Formel VI ist auch bekannt und wird in situ eingesetzt, indem man Dimethylsulfid mit Dimethylsulfat umsetzt (vgl. auch die o. g. Literaturstelle).

Die Azole der allgemeinen Formel III sind allgemein bekannte Verbindungen der organischen Chemie.

Zur Herstellung von physiologisch verträglichen Säureadditionssalzen der Verbindungen der allgemeinen Formel I kommen vorzugsweise folgende Säuren in Frage:

Die Halogenwasserstoffsäuren, wie z. B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Citronensäure, Salicylsäure, Sorbinsäure, Milchsäure, sowie Sulfonsäure, wie z. B. p-Toluolsulfonsäure und 1,5-Naphthalindisulfonsäure.

Die Säureadditionssalze der Verbindungen der allgemeinen Formel I können in einfacher Weise nach üblichen Salzbildungsmethoden, z. B. durch Lösen einer Verbindung der allgemeinen Formel I in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäßen verwendbaren Verbindungen der allgemeinen Formel I und ihre Säureadditionssalze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bisphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum, sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die obengenannten Erreger hervorgerufen werden.

Zur Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppsitorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder $^1/_2$, $^1/_3$ oder $^1/_4$ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin, und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit, und (i) Gleitmittel, z. B. Talkum, Calcium- und Magnesiumstearat, und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert, abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett, und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant,

Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös, appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objektes der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

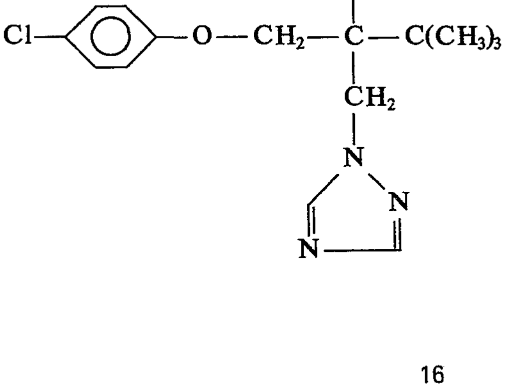

(I-1)

72,15 g (0,3 Mol) 2-(4-Chlorphenoxy-methyl)-2-tert.-butyl-oxiran und 24,15 g (0,35 Mol) 1,2,4-Triazol werden in 120 ml Ethanol 48 Stunden unter Rückfluß erhitzt. Anschließend wird eingeengt, der Rückstand in 200 ml Essigester aufgenommen und erhitzt. Danach wird im Eisbad abgekühlt, der Feststoff abgesaugt und mit Essigester nachgewaschen. Das Filtrat wird eingeengt, der Rückstand in Ether/Hexan gelöst und mit Chlorwasserstoff begast. Man saugt den Niederschlag ab, wäscht mit Ether nach und erhält durch Zugabe von Essigester/1 n Natronlauge die freie Base. Man erhält 60,2 g (65% der Theorie) 2-(4-Chlorphenoxy-methyl)-3,3-dimethyl-1-(1,2,4-triazol-1-yl)-butan-2-ol vom Schmelzpunkt 84 – 87° C.

Herstellung des Ausgangsproduktes

[Neues Zwischenprodukt der Formel (II)]

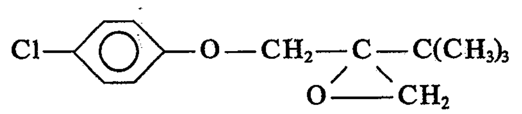

(II-1)

Eine Lösung von 189 ml (2,0 Mol) Dimethylsulfat in 1200 ml absolutem Acetonitril wird bei Raumtemperatur mit einer Lösung von 162 ml (2,2 Mol) Dimethylsulfid in 400 ml absolutem Acetonitril versetzt. Man läßt die Reaktionsmischung über Nacht bei Raumtemperatur rühren. Danach wird 118,8 g (2,2 Mol) Natriummethylat zugegeben. Man läßt 30 Minuten rühren und versetzt dann innerhalb von 30 Minuten tropfenweise mit einer Lösung von 272 g (1,2 Mol) 1-(4-Chlorphenoxy)-3,3-dimethyl-butan-2-on in 600 ml absolutem Acetonitril. Man läßt das Reaktionsgemisch über Nacht nachrühren. Anschließend wird eingeengt, der Rückstand zwischen Wasser und Essigester verteilt, die organische Phase abgetrennt, zweimal mit Wasser und einmal mit gesättigter Natriumchloridlösung gewaschen, über Natriumsulfat getrocknet, eingeengt und der Rückstand im Vakuum destilliert. Man erhält 242,4 g (84% der Theorie) 2-(4-Chlorphenoxy-methyl)-2-tert.-butyl-oxiran vom Siedepunkt 115 – 22° C/ 0,003 mm Hg-Säule und vom Schmelzpunkt 50 – 52° C.

Beispiel 2

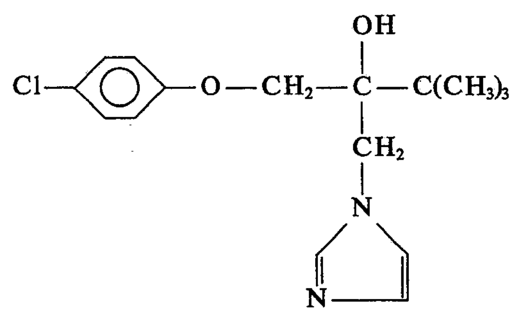

(I-2)

2,71 g (0,1178 Mol) Natrium in 250 ml absolutem Ethanol werden mit 8,02 g (0,1178 Mol) Imidazol versetzt. Innerhalb von 30 Minuten läßt man bei Raumtemperatur eine Lösung von 14,17 g (0,0589 Mol) 2-(4-Chlorphenoxy-methyl)-2-tert.-butyl-oxiran in 100 ml Ethanol zutropfen. Danach wird das Reaktionsgemisch 8 Stunden unter Rückfluß erhitzt, eingeengt und der Rückstand in Ether aufgenommen. Man extrahiert dreimal mit 1 n Salzsäure, neutralisiert die vereinigten Salzsäurephasen mit Natriumhydrogencarbonat und extrahiert anschließend mit Essigester. Nach dem Einengen und Umkristallisieren aus Cyclohexan erhält man 11,6 g (64% der Theorie) 2-(4-Chlorphenoxy-methyl)-3,3-dimethyl-1-(imidazol-1-yl)-butan-2-ol vom Schmelzpunkt 154 – 55° C.

Beispiel 3

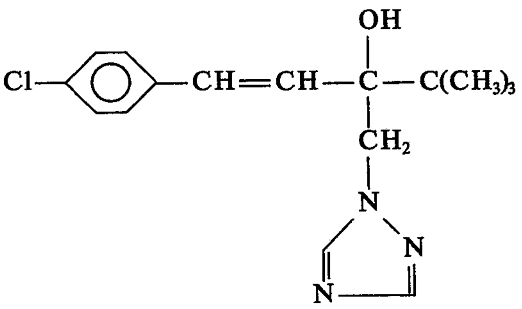

(I-3)

Eine Lösung von 17,75 g (0,075 Mol) 2-(4-Chlorphenyl-ethenyl)-2-tert.-butyl-oxiran und 6,9 g (0,1 Mol) 1,2,4-Triazol in 30 ml Ethanol werden 20 Stunden bei 150°C im Bombenrohr erhitzt. Danach wird das Reaktionsgemisch eingeengt und der kristalline Rückstand mit Ether verrührt. Der Feststoff wird anschließend abgesaugt und aus Acetonitril umkristallisiert. Man erhält 17,7 g (77% der Theorie) 1-(4-Chlorphenyl)-4,4-dimethyl-3-(imidazol-1-yl-methyl)-1-penten-3-ol vom Schmelzpunkt 139–41°C.

Beispiel 4

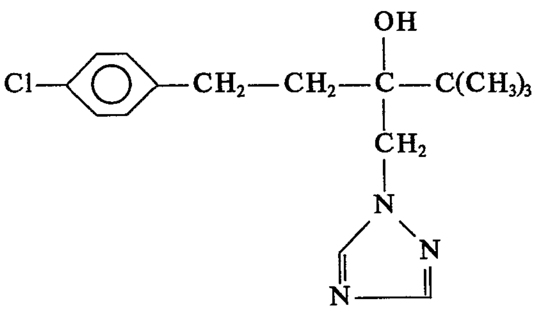

(I-4)

Eine Lösung von 17,9 g (0,075 Mol) 2-(4-Chlorphenyl-ethyl)-2-tert.-butyl-oxiran und 6,9 g (0,1 Mol) 1,2,4-Triazol in 30 ml Ethanol werden 20 Stunden bei 150°C im Bombenrohr erhitzt. Man läßt abkühlen und engt die Reaktionslösung ein. Der Rückstand wird in Ether gelöst, dreimal mit Wasser und einmal mit Natriumchloridlösung gewaschen, über Natriumsulfat getrocknet und eingeengt. Der Rückstand wird über eine Kieselgelsäule eingeengt (Laufmittel: Dichlormethan/Essigester 1 : 1). Man erhält 12,3 g (53,2% der Theorie) 1-(4-Chlorphenyl)-4,4-dimethyl-3-(1,2,4-triazol-1-yl-methyl)-pentan-3-ol als zähflüssiges Öl.

In analoger Weise wurden die nachfolgenden Verbindungen der allgemeinen Formel I

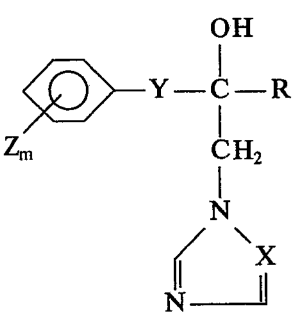

(I)

erhalten:

| Bsp. Nr. | $Z_m$ | Y | R | X | Schmelzpunkt (°C) |
|---|---|---|---|---|---|
| I- 5 | 4-Cl, 2-$CH_3$ | $-O-CH_2-$ | $-C(CH_3)_3$ | N | 125,5–29 |
| 6 | 2,4-$Cl_2$ | $-O-CH_2-$ | $-C(CH_3)_3$ | N | 120,5–23,5 |
| 7 | 4-$CH_3$ | $-O-CH_2-$ | $-C(CH_3)_3$ | N | 98–101,5 |

(Fortsetzung)

| Bsp. Nr. | $Z_m$ | Y | R | X | Schmelzpunkt (°C) |
|---|---|---|---|---|---|
| I- 8 | 2-$CH_3$ | $—O—CH_2—$ | $—C(CH_3)_3$ | N | 89–101 |
| 9 | 4-F | $—CH_2—CH_2—$ | $—C(CH_3)_3$ | N | 91–95,5 |
| 10 | 2-$CH_3$ | $—CH{=}CH—$ | $—C(CH_3)_3$ | N | Öl |
| 11 | 4-Cl | $—CH_2—CH_2—$ | $—C(CH_3)_3$ | N | 212 (Zers. (xHCl) |
| 12 | 2,4-$Cl_2$ | $—O—CH_2—$ | $—C(CH_3)_3$ | CH | 152–54 |
| 13 | 4-$CH_3$ | $—O—CH_2—$ | $—C(CH_3)_3$ | CH | 129–31 |
| 14 | 2-$CH_3$ | $—O—CH_2—$ | $—C(CH_3)_3$ | CH | 123–24 |
| 15 | 4-Cl, 2-$CH_3$ | $—O—CH_2—$ | $—C(CH_3)_3$ | CH | 157–59 |
| 16 | 4-Cl | $—CH_2—CH_2—$ | $—C(CH_3)_3$ | CH | 157,5–59,5 |
| 17 | 4-F | $—CH_2—CH_2—$ | $—C(CH_3)_3$ | CH | 124–25 |
| 18 | 2-$CH_3$ | $—CH_2—CH_2—$ | $—C(CH_3)_3$ | CH | 94–99 |
| 19 | 4-Cl | $—CH{=}CH—$ | $—C(CH_3)_3$ | CH | 158,5–62 |
| 20 | 4-F | $—CH{=}CH—$ | $—C(CH_3)_3$ | CH | 144–46 |
| 21 | 2-$CH_3$ | $—CH{=}CH—$ | $—C(CH_3)_3$ | CH | 127–32 |
| 22 | 4-Cl | $—O—CH_2—$ | 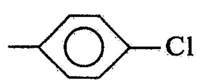 | CH | 216–17 · $\frac{1}{2}$ NDS+ |
| 23 | 4-$CH_3$ | $—CH{=}CH—$ | $—C(CH_3)_3$ | N | 117–19 |
| 24 | 4-$CH_3$ | $—CH{=}CH—$ | $—C(CH_3)_3$ | CH | 144–46 |
| 25 | 2,6-$Cl_2$ | $—CH{=}CH—$ | $—C(CH_3)_3$ | CH | 110–16 |
| 26 | 4-$CH_3$ | $—CH_2—CH_2$ | $—C(CH_3)_3$ | N | Öl |
| 27 | 2,4-$Cl_2$ | $—CH_2—CH_2—$ | $—C(CH_3)_3$ | CH | 118–19 |
| 28 | 4- 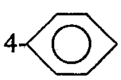 | $—O—CH_2—$ | $—C(CH_3)_3$ | CH | 169–70,5 |
| 29 | 2-Cl | $—O—CH_2—$ | $—C(CH_3)_3$ | CH | 122–24 |
| 30 | 2-Cl | $—O—CH_2—$ | $—C(CH_3)_3$ | N | 109–11 |
| 31 | 2,4-$Cl_2$ | $—CH_2—CH_2—$ | $—C(CH_3)_3$ | N | 94–95 |
| 32 | 2-$CH_3$ | $—CH_2—CH_2—$ | $—C(CH_3)_3$ | N | 82–83 |
| 33 | 4-Cl | $—O—CH_2—$ | 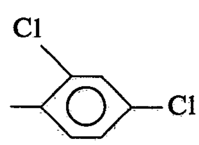 | CH | 134–35,5 |
| 34 | 4- 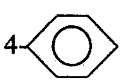 | $—O—CH_2—$ | $—C(CH_3)_3$ | N | 118–19,5 |
| 35 | 4-Cl | $—O—CH_2—$ | 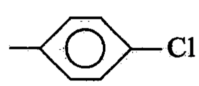 | N | 85–85 |
| 36 | 4-Cl | $—O—CH_2—$ | 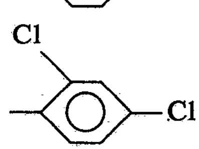 | N | 149–51 |
| 37 | 4-F | $—O—CH_2—$ | $—C(CH_3)_3$ | CH | 141–42 |
| 38 | 4-F | $—O—CH_2—$ | $—C(CH_3)_3$ | N | 73–75 |

(Fortsetzung)

| Bsp. Nr. | $Z_m$ | Y | R | X | Schmelzpunkt (°C) |
|---|---|---|---|---|---|
| I-39 | 3-Cl | $-O-CH_2-$ | $-C(CH_3)_3$ | CH | 124 |
| 40 | 2-Cl, 4-F | $-O-CH_2-$ | $-C(CH_3)_3$ | CH | 137 |
| 41 | 3-Cl | $-O-CH_2-$ | $-C(CH_3)_3$ | N | 72 |
| 42 | 2-Cl, 4-F | $-O-CH_2-$ | $-C(CH_3)_3$ | N | 130 |
| 43 | 3,4-$Cl_2$ | $-O-CH_2-$ | $-C(CH_3)_3$ | N | 124 |
| 44 | 4-$CH_3$ | $-CH_2-CH_2-$ | $-C(CH_3)_3$ | CH | 101–03 |
| 45 | 4-F | $-CH=CH-$ | $-C(CH_3)_3$ | N | 129–31 |
| 46 | 4-Cl | $-O-CH_2-$ | $-C(CH_3)_3$ | CH | 174–76 |
| 47 | 4-Cl | $-O-CH_2-$ | $-C(CH_3)_3$ | N | 109–11 |
| 48 | | $-O-CH_2-$ | $-C(CH_3)_3$ | N | 84–85 |
| 49 | 4-$OCH_3$ | $-O-CH_2-$ | $-C(CH_3)_3$ | N | 63–66 |
| 50 | 4-$C(CH_3)_3$ | $-O-CH_2-$ | $-C(CH_3)_3$ | N | 75–78 |
| 51 | 4-$OCF_3$ | $-O-CH_2-$ | $-C(CH_3)_3$ | N | $n_D^{20}$ = 1,4902 |

+ NDS = 1,5-Naphthalindisulfonsäure.

Entsprechend Beispiel 1 werden die nachfolgenden Zwischenprodukte der allgemeinen Formel II

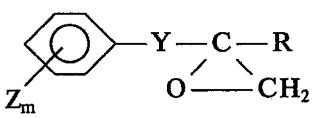

(II)

erhalten:

| Bsp. Nr. | $Z_m$ | Y | R | Siedepunkt (°C)/ mm Hg-Säule |
|---|---|---|---|---|
| II- 2 | 2,4-$Cl_2$ | $-O-CH_2-$ | $-C(CH_3)_3$ | 125–27/03 |
| 3 | 4-$CH_3$ | $-O-CH_2-$ | | 85/0,07 |
| 4 | 2-$CH_3$ | $-O-CH_2-$ | | 89/0,07 |
| 5 | 4-Cl, 2-$CH_3$ | $-O-CH_2-$ | | 114–17/0,33 |
| 6 | 4-Cl | $-CH_2-CH_2-$ | | 99–103/0,005 |
| 7 | 2,4-$Cl_2$ | $-CH_2-CH_2-$ | | 79/0,004 |
| 8 | 4-F | $-CH_2-CH_2-$ | | 79–89/0,003 |
| 9 | 4-$CH_3$ | $-CH_2-CH_2-$ | | 74–78/0,003 |
| 10 | 2-$CH_3$ | $-CH_2-CH_2-$ | | 95/0,005 |
| 11 | 4-Cl | $-CH=CH-$ | | Fp. 61–62,5 |
| 12 | 2,4-$Cl_2$ | $-CH=CH-$ | | nicht isoliert |
| 13 | 4-$CH_3$ | $-CH=CH-$ | | nicht isoliert |
| 14 | 4-F | $-CH=CH-$ | | 75/0,005 |
| 15 | 2-$CH_3$ | $-CH=CH-$ | | 71–74/0,01 |
| 16 | 2,6-$Cl_2$ | $-CH=CH-$ | | nicht isoliert |

## Verwendungsbeispiele

In den nachfolgenden Beispielen werden die nachstehend angegebenen Verbindungen als Vergleichssubstanzen eingesetzt:

A = 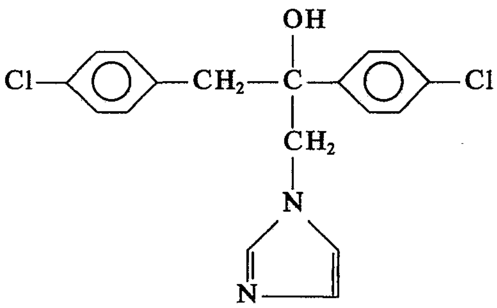

1,2-Bis(4-Chlorphenyl)-3-(imidazol-1-yl)-2-propanol

B = 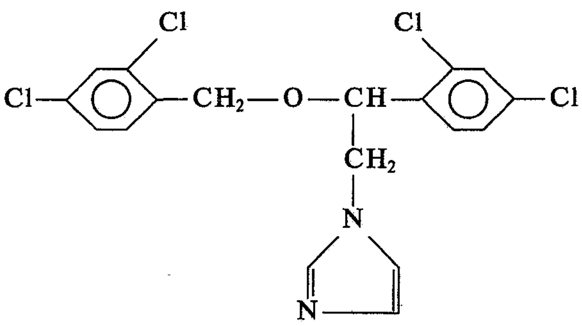

Miconazol

### Beispiel A

Antimykotische in-vitro-Wirksamkeit

Versuchsbeschreibung

Die in-vitro-Prüfung wurde im Reihenverdünnungstest mit Keiminokula von durchschnittlich $5 \times 10^4$ Keimen/ml Substrat durchgeführt. Als Nährmedium dienten

A) für Dermatophyten und Schimmelpilze:
Sabouraud's milieu d'epreuve
B) für Hefen:
Isotonic Sensitest-Broth von Oxid.

Die Bebrütungstemperatur betrug 18° C; Bebrütungszeit war 24 Stunden bei Hefen und 96 Stunden bei Dermatophyten und Schimmelpilzen.
In diesem Test zeigen beispielsweise die Beispiele 1, 2, 5, 6, 7, 9, 12, 15, 16, 17, 18 und 19 eine bessere antimykotische Wirkung als die aus dem Stand der Technik bekannte Verbindung (A).

### Beispiel B

Antimikrobielle in-vivo-Wirksamkeit (oral) bei Mäuse-Candidose

Versuchsbeschreibung

Mäuse vom Typ SPF-$CF_1$ wurden intravenös mit $1-2 \times 10^6$ logarithmisch wachsenden Candida-Zellen, die in physiologischer Kochsalzlösung suspendiert waren, infiziert. Eine Stunde vor und sieben Stunden nach der Infektion werden die Tiere mit jeweils 50 – 100 mg/kg Körpergewicht der Präparate oral behandelt.

Ergebnis

Unbehandelte Tiere starben 3 bis 6 Tage post infectionem. Die Überlebensrate am 6. Tag post infectionem betrug bei unbehandelten Kontrolltieren etwa 5%.
In diesem Test zeigen z. B. die erfindungsgemäßen Verbindungen 1, 6, 16 und 19 Wirkung bis sehr gute Wirkung, während die aus dem Stand der Technik bekannte Verbindung (B) keine Wirkung zeigt.

Beispiel C

Antimikrobielle in-vivo-Wirksamkeit (lokal) am Modell der experimentellen Meerschweinchen-Trichophytie

Versuchsbeschreibung

Weiße Meerschweinchen der Rasse Pribright-white wurden auf dem geschorenen, nicht skarifizierten Rücken mit einer Mikro- und Makrokonidien-Suspension von Trichophyton mentagrophytes infiziert. Die infizierten Tiere wurden, beginnend mit dem 3. Tag p.i., 1 × täglich mit 1%iger Lösung der erfindungsgemäßen Präparate (in Dimethylsulfoxid : Glycerin = 1 : 4) lokal behandelt.

Ergebnis

Bei unbehandelten Tieren entwickelt sich innerhalb von 12 Tagen p.i. das typische Bild einer Dermatophytose mit Rötung, Schuppen und Haarausfall bis zum totalen Integument-Defekt an der Infektionsstelle.
In diesem Test zeigen z. B. die erfindungsgemäßen Beispiele 1, 2, 6, 12 und 15 gute bis sehr gute Wirkung, d. h. keine Infektionszeichen am 12. bis 15. Tag p.i. bzw. lediglich geringe Rötung und vereinzelt Schuppen.

**Patentansprüche**

1. Antimykotisches Mittel, gekennzeichnet durch einen Gehalt an mindestens einem 1-Hydroxyethyl-azol-derivat der allgemeinen Formel I

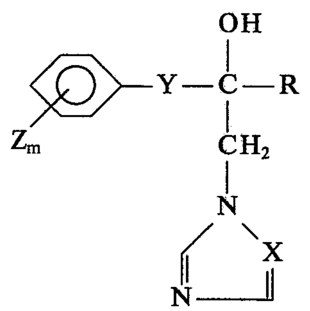

(I)

in welcher

R für geradkettiges oder verzweigtes Alkyl mit 1–4 Kohlenstoffatomen, gegebenenfalls durch Alkyl mit 1–2 Kohlenstoffatomen substituiertes Cycloalkyl mit 3–7 Kohlenstoffatomen sowie gegebenenfalls einfach oder mehrfach, gleich oder verschieden durch Halogen, Alkyl mit 1–4 Kohlenstoffatomen oder Halogenalkyl mit 1–2 Kohlenstoffatomen und 1–5 gleichen oder verschiedenen Halogenatomen substituiertes Phenyl steht,
X für ein Stickstoffatom oder die –CH-Gruppe steht,
Y für die Gruppierungen $-OCH_2-$, $-CH_2CH_2-$ oder $-CH=CH-$ steht,
Z für Halogen, geradkettiges oder verzweigtes Alkyl mit 1–4 Kohlenstoffatomen, Cycloalkyl mit 5–7 Kohlenstoffatomen, Alkoxy und Alkylthio mit jeweils 1–4 Kohlenstoffatomen, Halogenalkyl und Halogenalkoxy sowie Halogenalkylthio mit jeweils 1–2 Kohlenstoffatomen und 1–5 gleichen oder verschiedenen Halogenatomen sowie für jeweils gegebenenfalls durch Halogen oder Alkyl mit 1–4 Kohlenstoffatomen substituiertes Phenyl, Phenoxy, Phenylalkyl oder Phenylalkoxy mit jeweils 1–2 Kohlenstoffatomen im Alkylteil steht und
m für die Zahlen 0, 1, 2 oder 3 steht,

sowie deren physiologisch verträgliche Säureadditionssalze.

2. Antimykotisches Mittel, gekennzeichnet durch einen Gehalt an mindestens einem 1-Hydroxyethyl-azol-derivat der allgemeinen Formel I

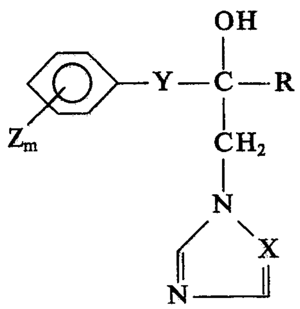

(I)

in welcher

R für tert.-Butyl, Isopropyl, Methyl, jeweils gegebenenfalls durch Methyl substituiertes Cyclopropyl, Cyclopentyl oder Cyclohexyl, oder für gegebenenfalls einfach oder zweifach durch Fluor, Chlor, Methyl oder Trifluormethyl substituiertes Phenyl steht,

Z für Fluor, Chlor, Brom, Methyl, tert.-Butyl, Cyclohexyl, Methoxy, Methylthio, Trifluormethyl, Trifluormethoxy, Trifluormethylthio sowie für jeweils gegebenenfalls einfach oder zweifach, durch Fluor, Chlor und Methyl substituiertes Phenyl, Phenoxy, Benzyl oder Benzyloxy steht, und

X, Y und m die in Anspruch 1 angegebene Bedeutung haben,

sowie deren physiologisch verträgliche Säureadditionssalze.

3. Verfahren zur Herstellung eines antimykotischen Mittels, dadurch gekennzeichnet, daß man 1-Hydroxyethyl-azol-derivate der allgemeinen Formel I in Anspruch 1 mit inerten, nichttoxischen, pharmazeutisch geeigneten Trägerstoffen vermischt.

4. Verfahren zur Herstellung eines antimykotischen Mittels, dadurch gekennzeichnet, daß man physiologisch verträgliche Säureadditionssalze von 1-Hydroxyethyl-azol-derivaten der allgemeinen Formel I in Anspruch 1 mit inerten, nichttoxischen, pharmazeutisch geeigneten Trägerstoffen vermischt.

## Claims

1. Antimycotic agent, characterised in that it contains at least one 1-hydroxyethyl-azole derivative of the general formula I

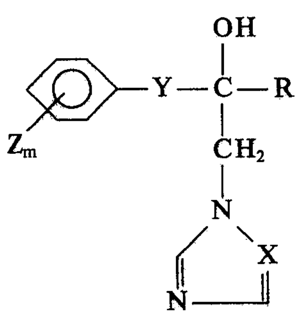

(I)

in which

R represents straight-chain or branched alkyl with 1–4 carbon atoms, cycloalkyl which has 3–7 carbon atoms and is optionally substituted by alkyl with 1–2 carbon atoms, or phenyl which is optionally monosubstituted or polysubstituted by identical or different substituents from the group comprising halogen, alkyl with 1–4 carbon atoms or halogenoalkyl with 1–2 carbon atoms and 1–5 identical or different halogen atoms,

X represents a nitrogen atom or the CH group,

Y represents the groups $-OCH_2-$, $-CH_2CH_2-$ or $-CH=CH-$,

Z represents halogen, straight-chain or branched alkyl with 1–4 carbon atoms, cycloalkyl with 5–7 carbon atoms, alkoxy or alkylthio with in each case 1–4 carbon atoms, halogenoalkyl, halogenoalkoxy or halogenalkylthio with in each case 1–2 carbon atoms and 1–5 identical or

different halogen atoms or phenyl, phenoxy, phenylalkyl or phenylalkoxy which each have 1–2 carbon atoms in the alkyl part and are each optionally substituted by halogen or alkyl with 1–4 carbon atoms and

m represents the numbers 0, 1, 2 or 3,

and physiologically acceptable acid addition salts thereof.

2. Antimycotic agent, characterised in that it contains at least one 1-hydroxyethyl-azole derivative of the general formula I

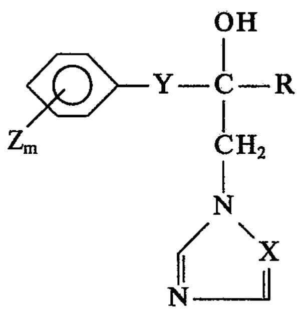

(I)

in which

R represents tert.-butyl, isopropyl, methyl, in each case optionally methyl-substituted cyclopropyl, cyclopentyl or cyclohexyl, or phenyl which is optionally monosubstituted or disubstituted by fluorine, chlorine, methyl or trifluoromethyl,

Z represents fluorine, chlorine, bromine, methyl, tert.-butyl, cyclohexyl, methoxy, methylthio, trifluoromethyl, trifluoromethoxy, trifluoromethylthio, or phenyl, phenoxy, benzyl or benzyloxy, in each case optionally monosubstituted or disubstituted by fluorine, chlorine or methyl, and

X, Y and m have the meaning indicated in Claim 1,

and physiologically acceptable acid addition salts thereof.

3. Process for the preparation of an antimycotic agent, characterised in that 1-hydroxyethyl-azole derivatives of the general formula I in Claim 1 are mixed with inert, non-toxic, pharmaceutically suitable excipients.

4. Process for the preparation of an antimycotic agent, characterised in that physiologically acceptable acid addition salts of 1-hydroxyethyl-azole derivatives of the general formula I in Claim 1 are mixed with inert, non-toxic, pharmaceutically suitable excipients.

**Revendications**

1. Composition antimycosique, caractérisée par une teneur en au moins un dérivé 1-hydroxyéthyl-azolique de formule générale I

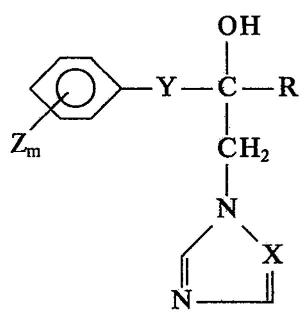

(I)

dans laquelle

R est un groupe alkyle linéaire ou ramifié, ayant 1 à 4 atomes de carbone, un groupe cycloalkyle ayant 3 à 7 atomes de carbone éventuellement substitué par un radical alkyle ayant 1 ou 2 atomes de carbone, ainsi qu'un groupe phényle éventuellement substitué une ou plusieurs fois par des substituants égaux ou différents tels qu'un halogène, un groupe alkyle ayant 1 à 4 atomes de carbone ou un groupe halogénalkyle ayant 1 ou 2 atomes de carbone et 1 à 5 atomes d'halogènes égaux ou différents,

X est un atome d'azote ou le groupe $-CH$,

Y représente les groupements $-OCH_2-$, $-CH_2CH_2-$ ou $-CH=CH-$,

Z est un halogène, un groupe alkyle linéaire ou ramifié ayant 1 à 4 atomes de carbone, un groupe cycloalkyle ayant 5 à 7 atomes de carbone, un groupe alkoxy et un groupe alkylthio ayant chacun 1 à 4 atomes de carbone, un groupe halogénalkyle et un groupe halogénalkoxy, de même qu'un groupe halogénalkylthio ayant chacun 1 ou 2 atomes de carbone et 1 à 5 atomes d'halogènes égaux ou différents, ainsi qu'un groupe phényle, phénoxy, phénylalkyle ou phénylalkoxy éventuellement substitués chacun par un halogène ou par un radical alkyle ayant 1 à 4 atomes de carbone, avec chacun 1 ou 2 atomes de carbone dans la partie alkyle, et

m représente les nombres 0, 1, 2 ou 3,

ainsi que leurs sels d'addition d'acides physiologiquement acceptables.

2. Composition antimycosique, caractérisée par une teneur en au moins un dérivé 1-hydroxyéthyl-azolique de formule générale I

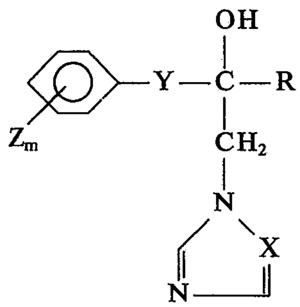

(I)

dans laquelle

R est un groupe tertio-butyle, isopropyle, méthyle, un groupe cyclopropyle, cyclopentyle ou cyclohexyle éventuellement substitués chacun par un radical méthyle, ou un groupe phényle éventuellement substitué une ou plusieurs fois par du fluor, du chlore, le radical méthyle ou le radical trifluorométhyle,

Z représente du fluor, du chlore, du brome, un groupe méthyle, tertio-butyle, cyclohexyle, méthoxy, mé hylthio, trifluorométhyle, trifluorométhoxy, trifluorométhylthio ainsi qu'un groupe phényle, phenoxy, benzyle ou benzyloxy éventuellement substitués chacun une ou plusieurs fois par du fluor, du chlore et un radical méthyle, et

X, Y et m ont la définition indiquée dans la revendication 1, ainsi que leurs sels d'addition d'acides physiologiquement acceptables.

3. Procédé de préparation d'une composition antimycosique, caractérisé en ce qu'on mélange des dérivés 1-hydroxyéthyl-azoliques de formule générale I suivant la revendication 1 avec des supports inertes non toxiques acceptables du point de vue pharmaceutique.

4. Procédé de préparation d'une composition antimycosique, caractérisé en ce qu'on mélange des sels d'addition d'acides physiologiquement acceptables de dérivés 1-hydroxyéthyl-azoliques de formule générale I suivant la revendication 1 avec des supports inertes non toxiques acceptables du point de vue pharmaceutique.